# FASCICULE DE BREVET EUROPEEN

(11) **EP 0 406 117 B1**
(45) Date de publication et mention de la délivrance du brevet: **03.05.1995**
(21) Numéro de dépôt: 90401871.0
(22) Date de dépôt: 28.06.1990
(51) Int. Cl.: C12N 1/20, C12R 1/225, C12R 1/25, C12R 1/46, A23K 1/00

(54) **Additif alimentaire microbien pour l'alimentation animale, et aliments le contenant**
Mikrobieller Futtermittelzusatz und dieser enthaltende Futtermittel
Microbial feed-additive for animals and feedstuffs containing the same

(30) Priorité: 30.06.1989 FR 8908778
(43) Date de publication de la demande: 02.01.1991
(73) Titulaire: FROMAGERIES BEL, F-75008 Paris (FR)
(72) Inventeur: Blanchet, Maurice, F-91430 Igny (FR); Dhoms, Philippe, F-41100 Vendôme (FR); Delespaul, Gilbert, F-41100 Vendôme (FR)
(74) Mandataire: Orès, Bernard

(56) Documents cités:
- CHEMICAL ABSTRACTS, vol. 110, 1989, page 621, résumé no. 6662d, Columbus, Ohio, US; & CS-A-249 286 (P. MICAN et al.) 15-02-1988
- JOURNAL OF THE SCIENCE OF FOOD AND AGRICULTURE, vol. 45, no. 3, 1988, pages 223-230, Society of Chemical Ind., Barking, Essex, GB; R. FULLER: "Principles of probiotic and antibiotic use in animal feeds"

## Description

La présente invention est relative à un nouvel additif alimentaire microbien et à son application comme facteur de croissance dans l'alimentation ainsi qu'aux aliments le contenant.

Il est connu que le développement extrêmement rapide de l'élevage à l'échelle industrielle a conduit à l'utilisation d'additifs spéciaux pour l'alimentation des animaux afin de restaurer leurs fonctions biologiques, modifiées par les nouveaux modes d'élevage.

Ces additifs alimentaires doivent d'une part, satisfaire aux besoins en micro-éléments, en macro-éléments et en vitamines des animaux élevés et nourris dans ces conditions intensives et leur assurer une prise de poids correcte et régulière ; ils doivent d'autre part, jouer un rôle de facteurs de croissance pour ces animaux dans les nouvelles conditions intensives d'élevage.

Parmi ces additifs utilisés, on peut citer notamment les antibiotiques ou autres agents chimiothérapeutiques, ainsi que les compositions microbiennes.

Ces différents facteurs ou promoteurs de croissance sont utilisés pour améliorer les performances zootechniques des animaux (mammifères, oiseaux, poissons) en leur assurant une meilleure régulation digestive.

Les antibiotiques, présentant un certain nombre d'inconvénients, notamment des effets résiduels, des phénomènes de résistance et/ou de toxicité, l'utilisation de compositions contenant des antibiotiques doit être limitée dans l'intérêt de la protection du consommateur.

C'est pourquoi les antibiotiques ont de plus en plus tendance à être substitués ou complétés par une autre classe de produits : les probiotiques, de plus en plus largement employés.

On entend par "probiotiques" des préparations sélectionnées, concentrées et revivifiables de bactéries lactiques ou autres germes aux propriétés nutritionnelles similaires ; ils renforcent les performances zootechniques des animaux et leur assurent une meilleure prévention des troubles digestifs.

Les probiotiques produisent leurs effets d'une part, en assurant la prophylaxie des agents pathogènes et d'autre part, en assurant un effet favorable sur la nutrition et l'augmentation de la valeur alimentaire. Comme exemples de compositions de ce type, on peut mentionnner les cultures vivantes, éventuellement lyophilisées de *Lactobacillus acidophilus*, *bulgaricus*, *casei*, *lactis*, *Bacillus subtilis* (brevet EP n° 287 699), de *Bacillus cereus* (brevet EP n° 296 051), de *Streptococcus faecium* (brevet EP n° 219 488), *Torulopsis*, *Aspergillus orizae*, *Streptomyces*, *Bifidobacterium sp.* avec des efficacités plus ou moins grandes sur les performances zootechniques des animaux.

Cependant, bien que les probiotiques utilisés dans l'Art antérieur soient capables d'éliminer les propriétés désavantageuses des antibiotiques, leur utilisation est accompagnée de certains inconvénients, notamment en ce qui concerne leur efficacité irrégulière ou leur difficulté de stockage et de conservation.

C'est pourquoi, la Demanderesse, afin de résoudre les problèmes posés par les probiotiques de l'Art antérieur, a isolé, sélectionné et produit des souches qui permettent d'obtenir des performances zootechniques nettement améliorées par rapport aux probiotiques de l'Art antérieur.

En effet, les souches de microorganismes mises au point par la Demanderesse ont l'avantage :
- d'augmenter la croissance des animaux,
- et/ou d'abaisser leur indice de consommation qui est définie comme la quantité d'aliment nécessaire à une unité de gain de poids,
- d'augmenter leur indice de transformation défini comme le gain de poids par unité d'aliment.

Les probiotiques proposés par la Demanderesse sont également faciles à administrer et d'une bonne aptitude à la conservation.

La présente invention s'est, en conséquence, donné pour but de pourvoir à de nouvelles souches de microorganismes et notamment de streptocoque et de lactobacilles, seuls ou en association, qui en tant que probiotiques, répondent mieux aux besoins de la pratique que les probiotiques de l'Art antérieur, notamment en ce qui concerne leurs performances zootechniques nettement améliorées.

La présente invention a pour objet une souche de *Streptococcus sp.* SLB, (ayant été déposée sous le n° I-841 auprès de la Collection Nationale des Cultures de microorganismes de l'Institut Pasteur,) caractérisée en ce que l'ADN de ladite bactérie présente notamment des sites de clivage par les enzymes de restriction EcoRV, Hind III, PstI, lesdites enzymes de restriction fournissant respectivement les fragments suivants :
- EcoRV : 2 fragments : 16,5 - 14,0 kbp ;
- Hind III : 10 fragments : 12,0 - 7,9 - 7,4 - 6,7 - 6,1 - 5,8 - 5,2 - 4,3 - 4,0 - 2,5 kbp ;
- PstI : 9 fragments : 9,5 - 8,3 - 6,8 - 6,3 - 5,7 - 4,7 - 3,9 - 2,2 - 1,9 kbp.

Ledit *Streptococcus sp.* SLB a été déposé auprès de la Collection Nationale des Cultures de microorganismes de l'Institut Pasteur sous le n° I-841.

La présente invention a également pour objet une souche de *Lactobacillus plantarum* I₃ (I-840), caractérisée en ce que son ADN présente notamment les sites de clivage par les enzymes de restriction BamHI, EcorI, EcoRV, KpnI, XhoI, lesdites enzymes de restriction fournissant respectivement les fragments suivants :
- BamHI : 5 fragments : 16,2 - 11,9 - 7,3 - 6,0 - 5,6 kbp ;
- EcoRI : 10 fragments : 7,1 - 6,6 - 5,6 - 5,5 - 4,9 - 3,7 - 3,4 - 2,4 - 2,1 - 0,9 kbp ;
- EcoRV : 3 fragments : 11,4 - 10,3 - 9,0 kbp ;
- KpnI : 3 fragments : 11,3 - 5,9 - 4,7 kbp ;
- XhoI : 4 fragments de 12,5 - 10,5 - 5,4 - 5,2 kbp.

Ledit *Lactobacillus* a été déposé auprès de la Collection Nationale des Cultures de microorganismes de l'Institut Pasteur sous le n° I-840.

La présente invention a également pour objet un additif alimentaire microbien, promoteur de croissance des animaux, caractérisé en ce qu'il comprend au moins la souche de *Streptococcus sp.* SLB conforme à l'invention, éventuellement associée à au moins une souche de lactobacille appropriée et éventuellement associée, en outre, à des supports, diluants et autres additifs, conservateurs adaptés à l'élevage des animaux.

Selon un mode de réalisation avantageux dudit additif, il comprend comme souche de lactobacille, la souche *Lactobacillus plantarum* I₃ conforme à l'invention.

Selon un autre mode de réalisation avantageux dudit additif conforme à l'invention, les proportions lactobacilles/streptocoques sont comprises entre 10:90 et 90:10.

Selon une disposition de ce mode de réalisation, ledit rapport est avantageusement compris entre 30:70 et 50:50.

La présente invention a également pour objet un aliment supplémenté pour animaux, caractérisé en ce qu'il contient un additif conforme à l'invention.

Selon un mode de réalisation avantageux dudit aliment, il contient entre 10⁵ et 10⁹ germes revivifiables par gramme d'aliment.

Les doses d'incorporation varient dans les fourchettes sus-citées selon l'espèce, l'âge, le niveau d'ingestion des animaux et dans une certaine mesure selon l'effet recherché.

Les aliments peuvent être présentés sous toutes les formes habituelles connues en élevage, simples ou composés, complets ou complémentaires.

On notera que l'eau de boisson pourra être un vecteur possible de ces probiotiques.

Outre les dispositions qui précèdent, l'invention comprend encore d'autres dispositions, qui ressortiront de la description qui va suivre, qui se réfère à des exemples de mise en oeuvre du procédé objet de la présente invention.

Il doit bien être entendu, toutefois, que ces exemples sont donnés uniquement à titre d'illustration de l'objet de l'invention, dont ils ne constituent en aucune manière une limitation.

### Exemple 1 : Caractéristiques morphologiques et biochimiques de Streptococcus SLB.

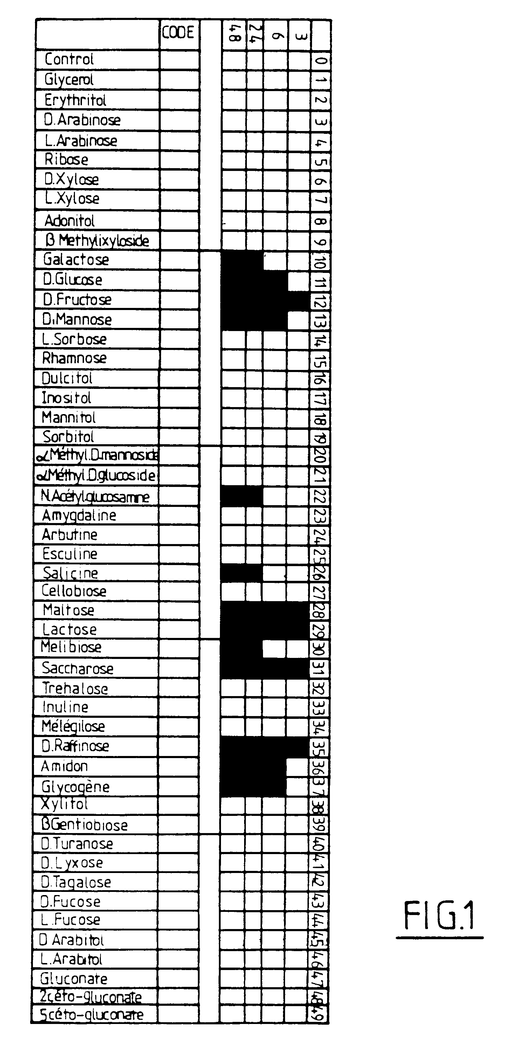

Ladite souche présente :
- les caractéristiques morphologiques suivantes :
   . cellules sphériques ou ovoïdes ayant un diamètre d'environ 2 »m, se présentant sous forme de paires, plus rarement de chaînettes,
   . gram + ,
   . non sporulé,
   . mobile - ,
   . anaérobie facultative,
   . catalase - ,
   . croissance à 10°C : - ,
   . croissance à 40°C : + ,
   . croissance à 45°C : + ,
   . croissance à 6,5 % NaCl : - ,
   . résistance à 63°C 30′ : + ,
   . croissance à 0,1 % bleu de méthylène : - , (milieu de Sherman).
- les caractéristiques biochimiques suivantes :
   . hémolyse sur gélose au sang de mouton : - ,
   . arginine dihydrolase : - ,
   . acétoïne : ± ,
   . culture sur lait tournesolé : réduction, acidification, coagulation (RAC),
Le résultat d'une galerie API 50 CH donnant le profil de fermentation des sucres : souche inoculée selon API avec une suspension de densité optique d'environ 1,0 à 1,5 mesurée à 550 nanomètres, soit à tube 5 dans l'échelle de Mac Farland est montré sur la figure 1.

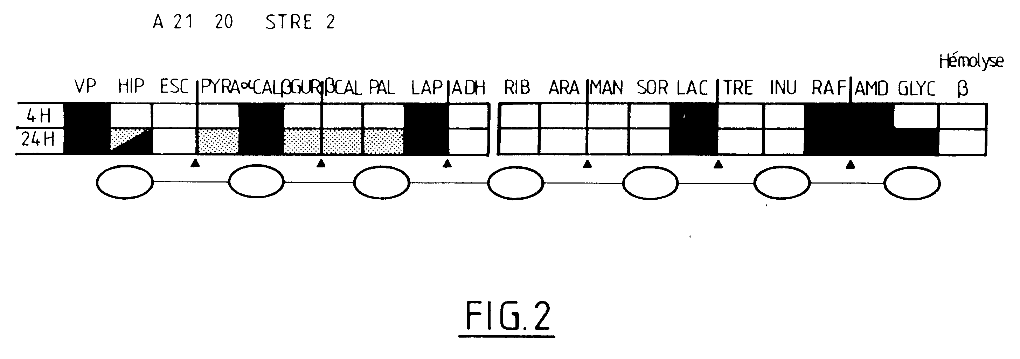

Le résultat d'une galerie 20 strept. (même technique d'inoculation) est donné sur la figure 2.

### Exemple 2 : Caractéristiques morphologiques et biochimiques de Lactobacillus plantarum I₃.

Ledit *Lactobacillus* conforme à l'invention présente :
- les caractéristiques morphologiques suivantes :
   . bâtonnet avec des extrémités arrondies, largeur de 0,9 à 1,2 »m,
   . gram + ,
   . non sporulé,
   . anaérobie facultatif,
   . mobilité - ,
   . pas de croissance à 45°C,
   . croissance à 15°C.
- les caractéristiques biochimiques suivantes :
   . arginine - ,
   . gaz - sur milieu Gibson et Abd-el-Malek,
   . résultat d'une galerie API 50 CH donnant le profil de fermentation des sucres : inoculation selon API avec une suspension de densité optique d'environ 0,5 à 0,75, mesurée à 550 nanomètres, soit tube 2 - 3 dans l'échelle de Mac Farland.

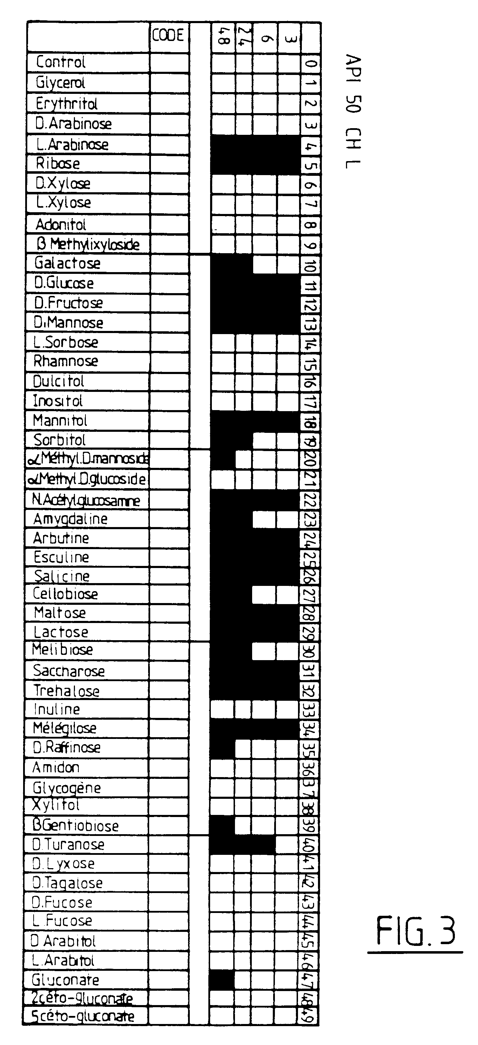

La figure 3 montre les résultats obtenus.

### Exemple 3 : Profil de restriction de StreptococcusSLB.

La méthodologie utilisée est celle décrite dans GRIMONT (Annales Institut Pasteur Microbiologie, 1986, 137B, 165-175) et comprend les étapes de
- clivage de l'ADN par 18 endonucléases de restriction : seules 3 endonucléases ont clivé l'ADN de *Streptococcus* SLB (EcoRV, Hind III, PstI) ;
- transfert de Southern des fragments clivés d'ADN obtenus sur membrane de nylon-nitrocellulose ;
- hybridation des fragments avec de l'ARN 16 + 23S d'*Escerichia Coli* marqué à l'extrémité de 5′ par du P radioactif ;
- lavage, l'autoradiographie et la réalisation d'une échelle de fragment de taille connue (marqueur Raoul I) par hybridation avec une sonde constituée par le plasmide pBR322 marqué au ³²P ;
- détermination des fragments visualisés des gènes ARN ribosomaux de la souche SLB par l'algorithme de Schaffer et Sederoff.

### Exemple 4 : Profil de restriction de Lactobacillus plantarum I3.

La méthodologie utilisée est celle décrite dans GRIMONT (Annales Institut Pasteur Microbiologie, 1986, 137B, 165-175) et comprend les étapes de
- clivage avec 18 endonucléases de restriction : seules 5 endonucléases ont clivé les ADN de *Lactobacillus plantarum* I3 : BamHI, EcoRI, EcoRV, KpNI, XhoI ;
- transfert de Southern des fragments clivés d'ADN obtenus sur membrane de nylon-nitrocellulose ;
- hybridation des fragments avec de l'ARN 16 + 23S d'*Escherichia Coli* marqué à l'extrémité de 5′ par du P radioactif ;
- lavage, l'autoradiographie et la réalisation d'une échelle de fragment de taille connue (marqueur Raoul I) par hybridation avec une sonde constituée par le plasmide pBR322 marqué au ³²P ;
- détermination des fragments visualisés des gènes ARN ribosomaux de la souche *Lactobacillus plantarum* I3 par l'algorithme de Schaffer et Sederoff.

### Exemple 5 : Effet probiotique du Streptocoque selon l'invention par rapport à un lot témoin et par rapport à d'autres streptocoques utilisés comme probiotiques.

Des lots de 15 veaux pesant 50 kg en moyenne à l'entrée de l'étable ont été nourris pendant 80 jours en moyenne avec respectivement :
- lot A : un aliment "veau" contenant environ 60 % de poudre de lait sans additif ni microbien, ni enzymatique, ni antibiotique ;
- lot B : un aliment "veau" contenant comme probiotique un *Streptococcus thermophilus* à raison de 10⁶ germes/gramme d'aliment ;
- lot C : un aliment "veau" contenant comme probiotique un *Streptococcus lactis* à raison de 10⁶ germes/gramme d'aliment ;
- lot D : un aliment contenant comme probiotique un *Streptococcus faecium* SF1 à raison de 10⁶ germes/gramme d'aliment ;
- lot E : avec *Streptococcus faecium* SF2 ;
- lot F : avec *Streptococcus* SF3 ;
- lot G : avec le *Streptococcus* SLB conforme à l'invention.

Le tableau I ci-après indique nettement l'effet "positif" sur le poids de carcasse moyen obtenu par les streptocoques (excepté le *Streptococcus faecium* SF3) par rapport au lot témoin et l'effet plus bénéfique du streptocoque SLB de l'invention par rapport aux autres streptocoques.

**TABLEAU I**

| SOUCHE | POIDS DE CARCASSE MOYEN OBTENU AVEC CETTE SOUCHE | POIDS DE CARCASSE MOYEN OBTENU AVEC LE TEMOIN **LOT A** | GAIN OU PERTE EN % |
|---|---|---|---|
| Streptocoque lactique "SLB" Lot G | 117,85 kg | 113,32 kg | + 4,0 % |
| *Streptococcus thermophilus* "M4" Lot B | 116,18 kg | 113,32 kg | + 2,53 % |
| *Streptococcus lactis* "SL 20" Lot C | 114,3 kg | 113,32 kg | + 0,85 % |
| *Streptococcus faecium* "SF 1" Lot D | 116,8 kg | 113,32 kg | + 3,07 % |
| *Streptococcus faecium* "SF 2" Lot E | 115,86 kg | 113,32 kg | + 2,24 % |
| *Streptococcus faecium* "SF 3" Lot F | 112,53 kg | 113,32 kg | - 0,7 % |

### Exemple 6 : Effet probiotique d'une association Streptococcus SLB selon l'invention et de Lactobacillus.

Des lots de 15 veaux pesant en moyenne 50 kg ont été nourris pendant 80 jours avec respectivement :
- lot A : aliment "veau" sans additif ni microbien, ni antibiotique ;
- lot B : aliment "veau" avec une association *streptococcus* SLB selon l'invention et un *Lactobacillus thermobacterium* dans des proportions lactobacillus/streptocoque 30/70 % et un taux d'incorporation dans l'aliment de 10⁶ germes/gramme d'aliment.

Les résultats sont présentés dans le tableau II ci-après.

**TABLEAU II**

| SOUCHES ASSOCIEES | POIDS DE CARCASSE OBTENU AVEC CES SOUCHES ASSOCIEES ET STREPT. SLB SEUL **LOT B** | POIDS DE CARCASSE MOYEN OBTENU AVEC LE TEMOIN **LOT A** | GAIN EN % |
|---|---|---|---|
| Streptocoque lactique sp "SLB" *Lactobacillus thermobactérium* "LT3" | 117,3 kg | 112,89 kg | +3,90% |
| Streptocoque "SLB" seul | 116,84 kg | 112,89 kg | +3,50% |

### Exemple 7 : Effet probiotique de l'association Streptococcus SLB/Lactobacillus plantarum I3 selon l'invention.

Des lots de 15 veaux pesant en moyenne 50 Kg ont été nourris pendant 80 jours avec respectivement :
- lot A : aliment "veau" sans additif ni microbien, ni enzymatique, ni antibiotique ;
- lot B : aliment "veau" avec une association *Streptococcus* SLB/*Lactobacillus plantarum* I3 à raison de 10⁶ germes/gramme d'aliment et une proportion *Lactobacillus plantarum* I3/*Streptococcus* SLB de 35/65.

Les résultats présentés sur le tableau III ci-après indiquent nettement l'effet bénéfique de cette association par rapport au témoin.

**TABLEAU III**

| SOUCHES ASSOCIEES | POIDS DE CARCASSE MOYEN OBTENU AVEC CES SOUCHES ASSOCIEES | POIDS DE CARCASSE MOYEN OBTENU AVEC LE TEMOIN | GAIN EN % |
|---|---|---|---|
| *Streptococcus* lactique sp "SLB" *Lactobacillus plantarum* I3 | 118,53 kg | 112,89 kg | + 5 % |

Il fait également nettement apparaître l'effet bénéfique de cette association particulière par rapport à une souche de *Streptococcus* SLB seul (exemple 5) et une association *Streptococcus* SLB/*Lactobacillus thermobacterium* (exemple 6).

### Exemple 8 : Effet probiotique du Streptococcus SLB sur les performances zootechniques des porcs à l'engrais.

Des lots de 32 porcs répartis en cases de 8 ont été nourris avec un aliment fourni en farine à base de blé jusqu'à des périodes de 75 jours d'engraissement. On a comparé les résultats zootechniques d'un lot témoin à un lot essai.
- lot A témoin : lot recevant uniquement de l'aliment sans additif ni microbien, ni enzymatique, ni antibiotique ;
- lot B : lot recevant de l'aliment dans lequel on a incorporé 1 % du *Streptococcus* SLB.

Le tableau IV ci-après donne les résultats obtenus et précise d'une part l'indice de consommation et d'autre part le gain de poids moyen quotidien (GMQ) en g/j.

**TABLEAU IV**

| | TEMOIN LOT A | AVEC STREPTOCOQUE LOT B |
|---|---|---|
| Indice de consommation | 2,89 | 2,81 (- 2,77 %) |
| GMQ en grammes/jour | 686 | 702 (+ 2,33 %) |

### Exemple 9 : Effet probiotique de l'association Lactobacillus plantarum I3/Streptococcus SLB sur les performances zootechniques des porcs à l'engrais.

Des lots de 32 porcs répartis en cases de 8 ont été nourris avec un aliment sous forme de farine à base de blé jusqu'à 75 jours d'engraissement.

On a comparé les résultats entre un lot témoin et un lot essai.
- lot A témoin : aliment sans probiotique, ni additif microbien, ni enzymatique ;
- lot B essai : aliment avec association de streptocoque SLB/*Lactobacillus plantarum* I3 à raison de 1 % et proportion du mélange *Lactobacillus plantarum* I3/streptocoque SLB : 30/70 %.

Le tableau V ci-après donne les résultats obtenus.

**TABLEAU V**

| | TEMOIN LOT A | ESSAI LOT B |
|---|---|---|
| Indice de consommation | 2,89 | 2,76 (- 4,5 %) |
| GMQ en grammes/jour | 686 | 713 (+ 3,94 %) |

### Exemple 10 : Procédé de préparation d'un additif conforme à l'invention.

On dilue à 10 % une poudre lyophilisée contenant au moins l'un des germes conforme à l'invention à 10¹⁰ dans un mélange lactosérum/levure lactique 50/50 et dans lequel on ajoute 1 % de silice hydratée comme agent anti-mottant.

### Exemple 11 : Exemple d'additif conforme à l'invention.

Aliment "veau" avec une association *Streptococcus* SLB/*Lactobacillus plantarum* I3 à raison de 10⁶ germes/gramme d'aliment et une proportion *Lactobacillus plantarum* I3/*Streptococcus* SLB de 35/65.

### Exemple 12 : Exemple d'aliment conforme à l'invention.

La composition d'un aliment est la suivante :

| | |
|---|---|
| - Poudre de lait écrémé | 26,9 % |
| - Poudre de lait réengraissé . Coprah | 45,0 % |
| - Amidon cru | 4,3 % |
| - Lactosérum | 10,8 % |
| - Farine de blé | 1,1 % |
| - Babeurre | 9,7 % |
| - CMV (Complément-Minéral-Vitamines) | 1,1 % |
| - Additif, facteur de croissance selon l'invention comprenant 70 % de *Lactobacillus plantarum* I3 et de *Streptococcus SLB* | 1,1 % |

## Revendications (Revendications pour l'(les) Etat(s) contractant(s) suivant(s): BE, DE, FR, IT, LU, NL)

1. Souche de *Streptococcus sp.* SLB, ayant été déposée sous le n° I-841 auprès de la Collection Nationale des Cultures de microorganismes de l'Institut Pasteur et présentant un effet probiotique.

2. Souche de *Lactobacillus plantarum* I₃, ayant été déposée sous le n° I-840 auprès de la Collection Nationale des Cultures de microorganismes de l'Institut Pasteur et présentant un effet probiotique.

3. Additif alimentaire microbien, promoteur de croissance des animaux, caractérisé en ce qu'il comprend au moins la souche de *Streptococcus sp.* SLB selon la revendication 1, éventuellement associée à au moins une souche de lactobacille appropriée et éventuellement associée, en outre, à des supports, diluants et autres additifs, conservateurs adaptés à l'élevage des animaux.

4. Additif selon la revendication 3, caractérisé en ce qu'il comprend comme souche de lactobacille, la souche *Lactobacillus plantarum* I₃ selon la revendication 2.

5. Additif selon la revendication 3 ou la revendication 4, caractérisé en ce que les proportions lactobacilles/streptocoques sont comprises entre 10:90 et 90:10.

6. Additif selon la revendication 5, caractérisé en ce que ledit rapport est avantageusement compris entre 30:70 et 50:50.

7. Aliment supplémenté pour animaux, caractérisé en ce qu'il contient un additif selon l'une quelconque des revendications 3 à 6.

8. Aliment selon la revendication 7, caractérisé en ce qu'il contient entre 10⁵ et 10⁹ germes revivifiables par gramme d'aliment.

## Revendications (Revendications pour l'(les) Etat(s) contractant(s) suivant(s): ES)

1. Procédé d'obtention d'une souche de *Streptococcus sp.*, caractérisé en ce que l'on prépare la souche de *Streptococcus sp.* SLB, déposée sous le n° I-841 auprès de la Collection Nationale des Cultures de microorganismes de l'Institut Pasteur et présentant un effet probiotique.

2. Procédé d'obtention d'une souche de *Lactobacillus plantarum*, caractérisé en ce que l'on prépare la souche de *Lactobacillus plantarum* I₃, déposée sous le n° I-840 auprès de la Collection Nationale des Cultures de microorganismes de l'Institut Pasteur et présentant un effet probiotique.

3. Procédé d'obtention d'un additif alimentaire microbien, promoteur de croissance des animaux, caractérisé en ce que l'on prépare un additif comprenant au moins la souche de *Streptococcus sp.* SLB obtenue selon la revendication 1, éventuellement associée à au moins une souche de lactobacille appropriée et éventuellement associée, en outre, à des supports, diluants et autres additifs, conservateurs adaptés à l'élevage des animaux.

4. Procédé d'obtention d'un additif selon la revendication 3, caractérisé en ce que la souche de lactobacille est la souche *Lactobacillus plantarum* I₃ obtenue selon la revendication 2.

5. Procédé d'obtention d'un additif selon la revendication 3 ou la revendication 4, caractérisé en ce que les proportions lactobacilles/streptocoques sont comprises entre 10:90 et 90:10.

6. Procédé d'obtention d'un additif selon la revendication 5, caractérisé en ce que ledit rapport est avantageusement compris entre 30:70 et 50:50.

7. Procédé d'obtention d'un aliment supplémenté pour animaux, caractérisé en ce que l'on ajoute un additif, obtenu selon l'une quelconque des revendications 3 à 6, à un aliment.

8. Procédé d'obtention d'un aliment selon la revendication 7, caractérisé en ce que l'on ajoute entre 10⁵ et 10⁹ germes revivifiables par gramme d'aliment.

## Claims (Claims for the following Contracting State(s): BE, DE, FR, IT, LU, NL)

1. *Streptococcus sp.* strain SLB, which was deposited under No. I-841 with the Collection Nationale des Cultures de microorganismes [National Collection of Microorganism Cultures] of the Pasteur Institute and displays a probiotic effect.

2. *Lactobacillus plantarum* strain I₃, which was deposited under No. I-840 with the Collection Nationale des Cultures de microorganismes of the Pasteur Institute and displays a probiotic effect.

3. Microbial feed additive which is an animal growth promoter, characterized in that it comprises at least the *Streptococcus sp.* strain SLB according to Claim 1, optionally combined with at least one suitable lactobacillus strain and optionally combined, in addition, with vehicles, diluents and other additives and preservatives suited to animal breeding.

4. Additive according to Claim 3, characterized in that it comprises as lactobacillus strain *Lactobacillus plantarum* strain I₃ according to Claim 2.

5. Additive according to Claim 3 or Claim 4, characterized in that the proportions of lactobacilli to streptococci are between 10:90 and 90:10.

6. Additive according to Claim 5, characterized in that the said ratio is advantageously between 30:70 and 50:50.

7. Supplemented animal feed, characterized in that it contains an additive according to any one of Claims 3 to 6.

8. Feed according to Claim 7, characterized in that it contains between 10⁵ and 10⁹ revivable microbes per gram of feed.

## Claims (Claims for the following Contracting State(s): ES)

1. Method for obtaining a *Streptococcus sp.* strain, characterized in that *Streptococcus sp.* strain SLB, deposited under No. I-841 with the Collection Nationale des Cultures de microorganismes [National Collection of Microorganism Cultures] of the Pasteur Institute and displaying a probiotic effect, is prepared.

2. Method for obtaining a *Lactobacillus plantarum* strain, characterized in that *Lactobacillus plantarum* strain I₃, deposited under No. I-840 with the Collection Nationale des Cultures de microorganismes of the Pasteur Institute and displaying a probiotic effect, is prepared.

3. Method for obtaining a microbial feed additive which is an animal growth promoter, characterized in that an additive is prepared comprising at least *Streptococcus sp.* strain SLB obtained according to Claim 1, optionally combined with at least one suitable lactobacillus strain and optionally combined, in addition, with vehicles, diluents and other additives and preservatives suited to animal breeding.

4. Method for obtaining an additive according to Claim 3, characterized in that the lactobacillus strain is *Lactobacillus plantarum* strain I₃ obtained according to Claim 2.

5. Method for obtaining an additive according to Claim 3 or Claim 4, characterized in that the proportions of lactobacilli to streptococci are between 10:90 and 90:10.

6. Method for obtaining an additive according to Claim 5, characterized in that the said ratio is advantageously between 30:70 and 50:50.

7. Method for obtaining a supplemented animal feed, characterized in that an additive obtained according to any one of Claims 3 to 6 is added to a feed.

8. Method for obtaining a feed according to Claim 7, characterized in that between 10⁵ and 10⁹ revivable microbes are added per gram of feed.

## Patentansprüche (Patentansprüche für folgende(n) Vertragsstaat(en): BE, DE, FR, IT, LU, NL)

1. Streptococcus sp. SLB-Stamm, dadurch **gekennzeichnet**, daß er unter der Nummer I-841 bei der Collection Nationale des Cultures de microorganismes de l'Institut Pasteur hinterlegt worden ist und eine probiotische Wirkung besitzt.

2. Lactobacillus plantarum I₃-Stamm, dadurch **gekennzeichnet**, daß er unter der Hinterlegungsnummer I-840 bei der Collection Nationale des Cultures de microorganismes de l'Institut Pasteur hinterlegt worden ist und eine probiotische Wirkung besitzt.

3. Mikrobieller Futtermittelzusatzstoff, der das Wachstum von Tieren fördert, dadurch **gekennzeichnet**, daß er mindestens den Streptococcus sp. SLB-Stamm nach Anspruch 1, gegebenenfalls zusammen mit mindestens einem geeigneten Lactobacillus-Stamm und gegebenenfalls weiterhin zusammen mit Trägern, Verdünnungsmitteln und anderen Zusatzstoffen, Konservierungsstoffen, die der Aufzucht von Tieren angepaßt sind, umfaßt.

4. Zusatzstoff nach Anspruch 3, dadurch **gekennzeichnet**, daß er als Lactobacillus-Stamm den Lactobacillus plantarum I₃-Stamm nach Anspruch 2 umfaßt.

5. Zusatzstoff nach Anspruch 3 oder 4, dadurch **gekennzeichnet**, daß die Anteile Lactobazillen/Streptokokken zwischen 10:90 und 90:10 liegen.

6. Zusatzstoff nach Anspruch 5, dadurch **gekennzeichnet**, daß das Verhältnis vorteilhafterweise zwischen 30:70 und 50:50 liegt.

7. Angereichertes Futtermittel für Tiere, dadurch **gekennzeichnet**, daß es einen Zusatzstoff nach einem der Ansprüche 3 bis 6 enthält.

8. Futtermittel nach Anspruch 7, dadurch **gekennzeichnet**, daß es zwischen 10⁵ und 10⁹ lebensfähigen Keimen pro Gramm Futtermittel enthält.

## Patentansprüche (Patentansprüche für folgende(n) Vertragsstaat(en): ES)

1. Verfahren zur Produktion eines Streptococcus sp.-Stammes, dadurch **gekennzeichnet**, daß man den Streptococcus sp. SLB-Stamm, der unter der Hinterlegungsnummer I-841 bei der Collection Nationale des Cultures de microorganismes de l'Institut Pasteur hinterlegt worden ist und eine probiotische Wirkung besitzt, herstellt.

2. Verfahren zur Produktion eines Lactobacillus plantarum-Stamms, dadurch **gekennzeichnet**, daß man den Lactobacillus plantarum I₃-Stamm, der unter der Hinterlegungsnummer I-840 bei der Collection Nationale des Cultures de microorganismes de l'Institut Pasteur hinterlegt worden ist und eine probiotische Wirkung besitzt, herstellt.

3. Verfahren zur Herstellung eines mikrobiellen Futtermittelzusatzstoffes, der das Wachstum von Tieren fördert, dadurch **gekennzeichnet**, daß man einen Zusatzstoff herstellt, der mindestens den Streptococcus sp. SLB-Stamm, erhalten nach Anspruch 1, gegebenenfalls zusammen mit mindestens einem geeigneten Lactobacillus-Stamm und gegebenenfalls weiterhin zusammen mit Trägern, Verdünnungsmitteln und anderen Zusatzstoffen, Konservierungsstoffen, die der Aufzucht von Tieren angepaßt sind, umfaßt.

4. Verfahren zur Herstellung eines Zusatzstoffes nach Anspruch 3, dadurch **gekennzeichnet**, daß der Lactobacillus-Stamm der Lactobacillus plantarum I₃-Stamm, erhalten gemäß Anspruch 2, ist.

5. Verfahren zur Herstellung eines Zusatzstoffes nach Anspruch 3 oder 4, dadurch **gekennzeichnet**, daß die Verhältnisse Lactobazillen/Streptokokken zwischen 10:90 und 90:10 liegen.

6. Verfahren zur Herstellung eines Zusatzstoffes nach Anspruch 5, dadurch **gekennzeichnet**, daß das Verhältnis vorteilhafterweise zwischen 30:70 und 50:50 liegt.

7. Verfahren zur Herstellung eines supplementierten Futtermittels für Tiere, dadurch **gekennzeichnet**, daß man einen Zusatzstoff, erhalten nach einem der Ansprüche 3 bis 6, einem Futtermittel zusetzt.

8. Vefahren zur Herstellung eines Futtermittels nach Anspruch 7, dadurch **gekennzeichnet**, daß man zwischen 10⁵ und 10⁹ lebensfähigen Keimen pro Gramm Futtermittel zusetzt.
